# EUROPEAN PATENT APPLICATION

(11) **EP 4 643 931 A1**
(43) Date of publication of application: **05.11.2025**
(21) Application number: 24883613.2
(22) Date of filing: 30.04.2024
(51) Int. Cl.: A61N 1/32, A61M 37/00

(54) **CONTROL DEVICE FOR RADIO FREQUENCY ELECTRODE ARRAY AND RADIO FREQUENCY THERAPEUTIC APPARATUS**

(30) Priority: 11.03.2024 CN 202410273947
(71) Applicant: Shenzhen Peninsula Medical Group, Shenzhen, Guangdong 518000 (CN)
(72) Inventor: LIANG, Yongsheng, Shenzhen, Guangdong 518000 (CN); CHEN, Yihui, Shenzhen, Guangdong 518000 (CN); ZHANG, Yuanchang, Shenzhen, Guangdong 518000 (CN); LEI, Xiaobing, Shenzhen, Guangdong 518000 (CN)
(74) Representative: Osterhoff, Utz
(86) International application number: PCT/CN2024/090957
(87) International publication number: WO 2025/189537

(57) **Abstract**

Disclosed are a controller for controlling a radio frequency (RF) electrode array and an RF treatment device having the same. The controller includes: a first subarray determination module, configured to determine a target RF electrode subarray in an initial output period from the RF electrode array; a second subarray determination module, configured to repetitively execute an operation of determining the target RF electrode subarray as a historical RF electrode subarray, and determining the target RF electrode subarray in a new output period from remaining RF electrode subarrays of the RF electrode array after excluding all historical RF electrode subarrays; and a control module, configured to control the target RF electrode subarray in a current output period to be in a first state to output RF energy, control the historical RF electrode subarray to be in a second state or a third state.

## Description

### CROSS-REFERENCE TO RELATED APPLICATIONS

The present application claims priority to Chinese Patent Application No. 202410273947.3, filed on March 11, 2024. The disclosures of the above-mentioned applications are incorporated herein by reference in their entireties.

### TECHNICAL FIELD

The present application relates to the technical field of medical apparatuses, and in particular to a controller for controlling a radio frequency (RF) electrode array and an RF treatment device having the same.

### BACKGROUND

The radio frequency (RF) electrode array includes a plurality of RF electrodes, which can be precisely positioned on the treatment area. Based on different treatment methods, the RF electrode array can be divided into two types, namely the minimally invasive RF electrode array and the noninvasive RF electrode array. Both two treatment methods can precisely deliver RF energy to the skin at a deep layer for treatment, aiming to stimulate the regeneration of skin collagen and dermis, thereby achieving skin tightening, wrinkle reduction, scar repair and other treatment effects.

In the related art, during the process of using the RF electrode to deliver RF energy to the skin at a deep layer, under the premise of the same total output power, when the target object of the RF energy outputted at each moment is the entire treatment area, the required treatment time is relatively long, which is easy to cause pain in the human body.

### SUMMARY

The main purpose of the present application is to provide a controller for controlling a radio frequency (RF) electrode array and an RF treatment device having the same, aiming to solve the problem that during the process of using the RF electrode to deliver RF energy to the skin at a deep layer, under the premise of the same total output power, when the target object of the RF energy output at each moment is the entire treatment area, the required treatment time is relatively long, which is easy to cause pain in the human body.

To achieve the above purpose, the present application provides a controller for controlling an RF electrode array, which is applied to an RF treatment device. The RF treatment device includes an RF generator and an RF electrode array electrically connected to the RF generator. The RF electrode array includes a plurality of RF electrode subarrays, and each RF electrode subarray includes at least one RF electrode. The controller includes:
a first subarray determination module, configured to determine a target RF electrode subarray in an initial output period from the RF electrode array;
a second subarray determination module, configured to repetitively execute an operation of determining the target RF electrode subarray as a historical RF electrode subarray, and determining the target RF electrode subarray in a new output period from remaining RF electrode subarrays of the RF electrode array after excluding all historical RF electrode subarrays until all of the RF electrode subarrays in the RF electrode array have been determined as the historical RF electrode subarrays; and
a control module, configured to control the target RF electrode subarray in a current output period to be in a first state to output RF energy during the current output period, control the historical RF electrode subarray to be in a second state or a third state, and control at least part of remaining RF electrode subarrays in the RF electrode array to be in the second state, an average power outputted by the RF electrode subarray in the first state being greater than an average power outputted by the RF electrode subarray in the second state, and the average power of the RF electrode subarray in the second state being greater than zero.

In an embodiment, the second subarray determination module includes:
a subarray selection unit, configured to determine all of the RF electrode subarrays that are not adjacent to the target RF electrode subarray in the current output period from the remaining RF electrode subarrays after excluding all historical RF electrode subarrays;
a second subarray determination unit, configured to determine the target RF electrode subarray in the new output period from all of the RF electrode subarrays that are not adjacent to the target RF electrode subarray in the current output period; and
a repetitive control unit, configured to repetitively execute the subarray selection unit and the second subarray determination unit in sequence until all of the RF electrode subarrays in the RF electrode array have been determined as the historical RF electrode subarrays.

In an embodiment, the control module is further configured to control the target RF electrode subarray to be in the second state in a next output period, during the current output period.

In an embodiment, the control module includes:
a first control unit, configured to control the target RF electrode subarray in the current output period to be in the first state to output RF energy during the current output period, control the historical RF electrode subarray to be in the second state or the third state, and further control at least part of remaining RF electrode subarrays in the RF electrode array to be in the second state;
an output accumulation unit, configured to accumulate a cumulative RF energy value outputted by each RF electrode subarray from a first historical output period to a current output period; and
a second control unit, configured to control each RF electrode subarray. In response to that the cumulative RF energy value is greater than or equal to a preset RF energy threshold, the second control unit is configured to control the RF electrode subarray to be in a power-free output state.

In an embodiment, the first control unit includes:
a temperature parameter acquisition subunit configured to acquire a real-time temperature parameter collected by a temperature sensor; and
an output power adjustment subunit configured to adjust a real-time output power of the target RF electrode subarray in the current output period according to the real-time temperature parameter.

In an embodiment, the target RF electrode subarray in the current output period includes at least two RF electrode subarrays that are not adjacent with each other.

In an embodiment, each of the target RF electrode subarray has equal number of RF electrodes, or a difference between numbers of RF electrodes in each target RF electrode subarray is less than a preset number threshold.

In an embodiment, an output power of the RF electrode subarray in the second state is less than or equal to 40% of an output power of the RF electrode subarray in the first state.

In an embodiment, an output power of the RF electrode subarray in the second state is less than or equal to 40% of an output power of the RF electrode subarray in the first state.

In an embodiment, the RF treatment device includes an RF generator, an RF electrode array, and the controller for controlling the RF electrode array as mentioned in the above embodiments.

In an embodiment, the RF generator includes a plurality of separately controllable RF sub-generators, and each RF sub-generator is configured to control the target RF electrode subarray in different output periods.

In an embodiment, the RF treatment device includes a monopolar mode, and all of the RF electrodes in one RF electrode subarray have same electrical polarities in the monopolar mode.

In an embodiment, the RF treatment device includes a bipolar mode, and one RF electrode subarray includes at least two RF electrodes with opposite electrical polarities in the bipolar mode.

In the controller for controlling the RF electrode array of the present application, the RF electrode array is divided into a plurality of RF electrode subarrays. The first subarray determination module selects one or more RF electrode subarrays as target RF electrode subarrays in an initial output period from a plurality of RF electrode subarrays. The control module controls the target RF electrode subarray in the initial output period to output RF energy in a first state, and controls at least part of the remaining RF electrode subarrays in the RF electrode array after excluding the target RF electrode subarray in the initial output period to be in a second state. While or after the target RF electrode subarray in the initial output period is determined, the second subarray determination module repetitively executes an operation of determining the target RF electrode subarray as a historical RF electrode subarray, and determines the target RF electrode subarray in a new output period from remaining RF electrode subarrays of the RF electrode array after excluding all historical RF electrode subarrays until all of the RF electrode subarrays in the RF electrode array have been determined as the historical RF electrode subarrays. For each new output period, when the current output period is the new output period, the control module is configured to control the target RF electrode subarray in the new output period to output RF energy in the first state, control at least part of remaining RF electrode subarrays in the RF electrode array after excluding the target RF electrode subarray in the current output period and the historical RF electrode subarray to be in the second state, and control the historical RF electrode subarray to be in the second state or the third state. It is can be seen that, during the process of using the RF electrode to deliver RF energy to the skin at a deep layer, under the premise that the total output energy required by per unit treatment area (namely the RF electrode subarray) is the same, the present application can reduce the instantaneous treatment area at each moment and reduce the instantaneous pain by performing time-sharing treatment on each local area of the entire treatment area. In addition, at least part of the RF electrode subarrays in the present application have output RF energy in the first state, and have also output RF energy in the second state. Through the energy accumulation of the low-power energy output in the second state, the energy time required to achieve the treatment purpose by only outputting RF energy in the first state with high power is partially reduced, thereby shortening the overall treatment time and avoiding the local area from causing pain to the human body due to excessively long treatment time. In this way, the problem that under the premise of the same total output power, when the target object of the RF energy outputted at each moment is the entire treatment area, the treatment time is long, which is easy to cause pain to the human body. In addition, during the entire treatment process, through the RF electrode subarray outputting the low RF energy in the second state with low power or other means, not only heat diffusion in the treatment area can be generated, and the treatment uniformity can be improved. Further, the treatment area can be expanded without increasing the pain, thereby improving the treatment effectiveness.

### BRIEF DESCRIPTION OF THE DRAWINGS

FIG. 1 is a schematic structural diagram of a controller for controlling a radio frequency (RF) electrode array according to an embodiment of the present application.
FIG. 2 is a schematic structural diagram of an RF treatment device according to an embodiment of the present application.
FIG. 3 is a schematic structural diagram of an RF electrode array in the monopolar mode according to an embodiment of the present application.
FIG. 4 is a schematic structural diagram of the RF electrode array in the bipolar mode according to an embodiment of the present application.

10, first subarray determination module; 20, second subarray determination module; 30, control module; 40, RF generator; 50, RF electrode array.

The realization of the objective, functional characteristics, and advantages of the present application are further described with reference to the accompanying drawings.

### DETAILED DESCRIPTION OF THE EMBODIMENTS

In order to make the purpose, technical solutions and advantages of the present application clearer, the technical solution in the embodiment of the present application will be clearly and completely described below in conjunction with the drawings of embodiments in the present application. Obviously, the described embodiments are only part of the embodiments of the present application, not all of the embodiments. Based on the embodiments in the present application, all other embodiments obtained by those skilled in the art without creative work shall fall within the scope of the present application.

It should be noted that in the present application, the terms "comprise", "include" or any other variants thereof are intended to cover a non-exclusive inclusion. Thus, a process, method, article, or system that includes a series of elements not only includes those elements, but also includes other elements that are not explicitly listed, or also includes elements inherent to the process, method, article, or system. If there are no more restrictions, the element defined by the sentence "including a..." does not exclude the existence of other identical elements in the process, method, article or system that includes the element.

In the present application, unless otherwise clearly specified and limited, the terms "connected", "fixed", etc. should be interpreted broadly. For example, "fixed" can be a fixed connection, a detachable connection, or a whole; can be a mechanical connection or an electrical connection; may be directly connected, or indirectly connected through an intermediate medium, and may be the internal communication between two elements or the interaction relationship between two elements, unless specifically defined otherwise. In addition, in the present application, suffixes such as "module", "component" or "unit" used to represent elements are only configured to facilitate the description of the present application and have no specific meaning in themselves. Therefore, "module", "component" or "unit" can be used in a mixed manner.

For those skilled in the art, the specific meaning of the above-mentioned terms in the present application can be understood according to specific circumstances. In addition, the technical solutions of the various embodiments can be combined with each other, but the combinations must be based on the realization of those skilled in the art. When the combination of technical solutions is contradictory or cannot be achieved, it should be considered that such a combination of technical solutions does not exist, nor does it fall within the scope of the present application.

In the related art, using the radio frequency (RF) electrode array for treatment is a safe, efficient and accurate skin treatment method that can help people improve skin quality. In an embodiment, the RF electrode array can be divided into two types, namely the minimally invasive RF electrode array and the noninvasive RF electrode array. The minimally invasive RF electrode array is the RF microneedle array, which uses an RF microneedle as the RF electrode. The microneedle head can penetrate the skin surface and accurately deliver RF energy to the deep tissues of the skin, avoiding the side effects that may occur in conventional RF treatments. In the noninvasive RF electrode array, the RF head directly attaches to the skin surface, and uses the RF electrode to build a current path without damaging the skin, thereby performing treatment by passing the current through the deep tissues of the skin. In order to achieve the expected treatment effect, in the process of using the RF electrode to deliver RF energy to the deep layers of the skin, under the premise of the same total output power, when the target object of the RF energy outputted at each moment is the entire treatment area, the treatment time is long, which is easy to cause pain in the human body. Pain is the result of a combination of a plurality of factors, which is positively correlated with the treatment time, treatment area, and power. For whole-surface treatment (namely, the target object of the RF energy outputted at each moment is the entire treatment area), the treatment area and treatment time are important factors in causing pain compared to single-point treatment (namely, the target object of the RF energy outputted at each moment is the local treatment area).

Therefore, the present application provides following technical solutions. The RF electrode array is divided into a plurality of RF electrode subarrays. The first subarray determination module selects one or more RF electrode subarrays as target RF electrode subarrays in an initial output period from a plurality of RF electrode subarrays. The control module controls the target RF electrode subarray in the initial output period to output RF energy in a first state, and controls at least part of the remaining RF electrode subarrays in the RF electrode array after excluding the target RF electrode subarray in the initial output period to be in a second state. While or after the target RF electrode subarray in the initial output period is determined, the second subarray determination module repetitively executes an operation of determining the target RF electrode subarray as a historical RF electrode subarray, and determines the target RF electrode subarray in a new output period from remaining RF electrode subarrays of the RF electrode array after excluding all historical RF electrode subarrays until all of the RF electrode subarrays in the RF electrode array have been determined as the historical RF electrode subarrays. For each new output period, when the current output period is the new output period, the control module is configured to control the target RF electrode subarray in the new output period to output RF energy in the first state, control at least part of remaining RF electrode subarrays in the RF electrode array after excluding the target RF electrode subarray in the current output period and the historical RF electrode subarray to be in the second state, and control the historical RF electrode subarray to be in the second state or the third state. That is, the control module is configured to control the target RF electrode subarray in a current output period (which can be divided into two situations, an initial period and a new output period, according to the above description) to output RF energy in the first state, control the historical RF electrode subarray (if any) to be in a second state or a third state, and control at least part of remaining RF electrode subarrays in the RF electrode array after excluding the target RF electrode subarray in the current output period and the historical RF electrode subarray to be in the second state.

It is can be seen that, during the process of using the RF electrode to deliver RF energy to the skin at a deep layer, under the premise that the total output energy required by the RF electrode subarray is the same, at least part of the RF electrode subarrays in the present application have output RF energy in the first state, and have also output RF energy in the second state. The present application can reduce the instantaneous treatment area at each moment and reduce the instantaneous pain by performing time-sharing treatment on each local area of the entire treatment area. In addition, through the energy accumulation of the low-power energy output in the second state, the energy time required to achieve the treatment purpose by only outputting RF energy in the first state with high power is partially reduced, thereby shortening the overall treatment time and avoiding the local area from causing pain to the human body due to excessively long treatment time. In this way, the problem that under the premise of the same total output power, when the target object of the RF energy outputted at each moment is the entire treatment area, the treatment time is long, which is easy to cause pain to the human body. In addition, during the entire treatment process, through the RF electrode subarray outputting the low RF energy in the second state with low power or other means, not only the working time of the RF energy outputted in the first state can be reduced, but also heat diffusion in the treatment area can be generated, and the treatment uniformity can be improved. Further, the treatment area can be expanded without increasing the pain, thereby improving the treatment effectiveness.

The inventive concept of the present application is further described below in conjunction with some specific embodiments.

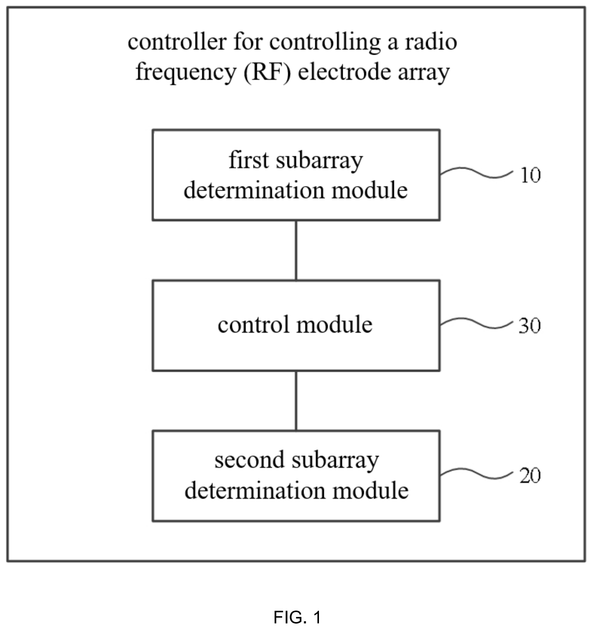

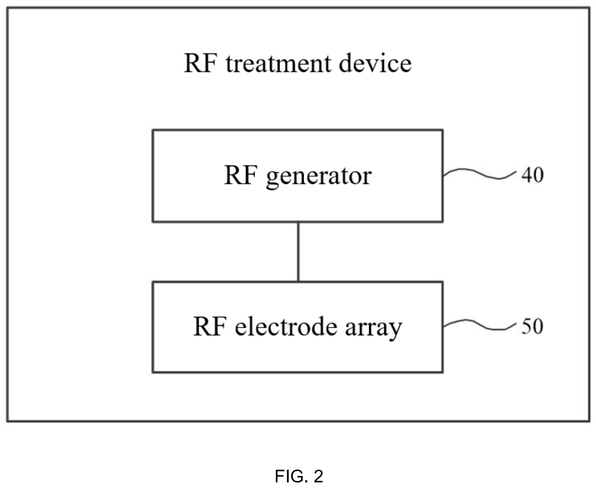

As shown in FIG. 1, in the first aspect, the present application provides an embodiment of a controller for controlling an RF electrode array. As shown in FIG. 1 and FIG. 2, FIG. 1 is a schematic structural diagram of the controller for controlling the RF electrode array according to a first embodiment of the present application, and FIG. 2 is a schematic structural diagram of an RF treatment device applied by the controller for controlling the RF electrode array of the present application.

In this embodiment, the controller for controlling the RF electrode array is applied to the RF treatment device which includes an RF generator 40 and an RF electrode array 50 electrically connected to the RF generator. The RF electrode array 50 includes a plurality of RF electrode subarrays, and each RF electrode subarray includes at least one RF electrode.

It should be understood that there may be only one RF generator 40, that is, one single RF generator 40 supplies power to the RF electrode array 50. The RF generator 40 may also be composed of a plurality of RF sub-generators that can be controlled separately. Each RF sub-generator may independently control the connection or disconnection of one or more RF electrode subarrays, and control their output power.

It can be understood that the RF electrode array 50 includes a series of mutually coupled, tiny RF electrodes arranged according to certain rules. These RF electrodes are usually made of insulating materials and metals. The metal part is configured to deliver RF energy to the skin at a deep layer. This arrangement of RF electrodes can ensure that the RF energy is evenly and accurately delivered to the skin during the treatment process, thereby stimulating the collagen production and the regeneration and reconstruction of the skin. It is worth mentioning that the RF electrode array 50 can be provided at different positions of the RF treatment device, which depends on the device design. Usually, the RF electrode array 50 is disposed at the treatment end of the handle of the RF treatment device.

It should be understood that relevant personnel can divide the RF electrodes in the RF electrode array 50 in advance to obtain a plurality of RF electrode subarrays, with the RF electrode subarray as the minimum output unit. For the RF electrode device, its working mode can be divided into a monopolar mode and a bipolar mode. If the RF electrode device works in the monopolar mode, the minimum number of RF electrodes in each RF electrode subarray can be one. If the RF electrode device works in the bipolar mode, the minimum number of RF electrodes in each RF electrode subarray can be two, that is, two RF electrodes with opposite electrical polarities (one electrical polarity is positive and one electrical polarity is negative). Of course, the number of RF electrodes greater than the minimum number above, such as three or four, can also be provided in the RF electrode subarray.

The controller for controlling the RF electrode array includes a first subarray determination module 10, a second subarray determination module 20, and a control module 30, which are described in detail below.

The first subarray determination module 10 is configured to determine the target RF electrode subarray in the initial output period from the RF electrode array 50.

In an embodiment, the initial output period refers to the output period when the RF electrode array 50 starts working, and the target RF electrode subarray is the RF electrode subarray in the RF electrode array 50. Determining the target RF electrode subarray in the initial output period from the RF electrode array 50 is to determine the RF electrode subarray that outputs RF energy in the first state from the RF electrode array 50 when the RF electrode array 50 starts working, and at least part of the remaining RF electrode subarrays in the RF electrode array 50 output RF energy in the second state.

In an embodiment, each of the target RF electrode subarray has equal number of RF electrodes, or a difference between numbers of RF electrodes in each target RF electrode subarray is less than a preset number threshold, so as to control the uniformity of the treatment energy distribution. The difference in the number of RF electrodes in the target RF electrode subarray is usually related to the total number of RF electrodes in the RF electrode array 50, but in general, the difference in number needs to be controlled to be no greater than 5 (namely, the preset number threshold is 5) to avoid a large difference in the area and power density (power per unit area) of instantaneous treatment.

It can be understood that at the beginning of treatment, all of the RF electrode subarrays are available, that is, they are all RF electrode subarrays to be selected. In this embodiment, a random selection method is used to determine the target RF electrode subarray in the initial output period from the RF electrode array 50. Of course, it can be understood that the first subarray determination module 10 can also use other methods to determine the target RF electrode subarray in the initial output period, such as a look-up table, and the like. The present application does not limit the determination method of the target RF electrode subarray in the initial output period.

The target RF electrode subarray takes the RF electrode subarray as a selection object, and in each output period, the RF electrode subarray selected as the target RF electrode subarray outputs RF energy in a first state. For a single output period, the target RF electrode subarray may include one or more RF electrode subarrays. When the target RF electrode subarray includes a plurality of RF electrode subarrays, at least two RF electrode subarrays are not adjacent to each other to prevent the RF electrode subarrays from bordering and merging into a larger treatment area to produce more pain that is easily perceived. By determining a plurality of nonadjacent RF electrode subarrays as target RF electrode subarrays, the number of times all of RF electrode subarrays in RF electrode array 50 have been determined as the historical RF electrode subarrays can be reduced under the premise that the human body does not feel pain during treatment. That is, the number of times the second subarray determination module 20 triggers the control module 30 is reduced, thereby reducing the time to finish the treatment. Moreover, when the total treatment area is the same, the total treatment area composed of a plurality of nonadjacent treatment areas (namely, RF electrode subarrays) causes less pain to the human body than the total treatment area composed of adjacent treatment areas.

The second subarray determination module 20 is used for repetitively configuring the target RF electrode subarray as the historical RF electrode subarray, and determining the target RF electrode subarray in the new output period from the remaining RF electrode subarrays of the RF electrode array 50 after excluding all the historical RF electrode subarrays, until all the RF electrode subarrays in the RF electrode array 50 have been determined as the historical RF electrode subarrays.

It should be understood that, during a treatment process, only certain RF energy are required to be received by the skin area treated by the RF electrode array 50. Based on this, when determining the target RF electrode subarray that subsequently outputs RF energy in the first state, the remaining RF electrode subarrays of the RF electrode array 50 after excluding all historical RF electrode subarrays can be selected, thereby avoiding excessive RF energy delivered to the same skin area and avoiding adverse effects on the human body. It should be noted that, in this embodiment, the second subarray determination module 20 can be directly connected to the first subarray determination module 10, or the second subarray determination module 20 is connected to the first subarray determination module 10 through the control module 30, to obtain the target RF electrode subarray in the initial output period, thereby determining the target RF electrode subarray in the new output period. Each new output period corresponds to a target RF electrode subarray, and the target RF electrode subarray outputs RF energy in the first state during its corresponding output period.

In addition, it should be understood that the second subarray determination module 20 can determine a plurality of new output periods in the circular execution process. For each new output period, RF energy outputted by the target RF electrode subarray in the new output period only act on partial skin treatment area. It can be understood that, by determining all of the RF electrode subarrays that are not adjacent to the target RF electrode subarrays in the current output period from the remaining RF electrode subarrays of the RF electrode array 50 after excluding all historical RF electrode subarrays, and determining the target RF electrode subarray in the new output period from all of the RF electrode subarrays that are not adjacent to the target RF electrode subarrays in the current output period, obvious pain in the local skin caused by the proximity of the skin areas treated in two consecutive output periods can be avoided. In this embodiment, the target RF electrode subarray in each new output period is determined, so that in the corresponding output period, the corresponding target RF electrode subarray outputs RF energy in the first state, all historical RF electrode subarrays are working in the second state or the third state, and at least part of the remaining RF electrode subarrays in the RF electrode array 50 are working in the second state, thereby treating all areas in the skin and achieving the treatment purpose.

In an embodiment, the second subarray determination module 20 includes a subarray selection unit, a second subarray determination unit, and a repetitive control unit.

The subarray selection unit is configured to determine all of the RF electrode subarrays that are not adjacent to the target RF electrode subarray in the current output period from the remaining RF electrode subarrays after excluding all historical RF electrode subarrays.

The second subarray determination unit is configured to determine the target RF electrode subarray in the new output period from all of the RF electrode subarrays that are not adjacent to the target RF electrode subarray in the current output period.

The repetitive control unit is configured to repetitively execute the subarray selection unit and the second subarray determination unit in sequence until all RF electrode subarrays in the RF electrode array 50 have been determined as the historical RF electrode subarrays.

In general, a complete treatment period includes a plurality of output periods, for example, there are output periods, each output period corresponds to a target RF electrode subarray that outputs RF energy in the first state. That is, target RF electrode subarrays will be determined, and the initial output period is the first output period. The target RF electrode subarray in the first output period is the first target RF electrode subarray. The first target RF electrode subarray outputs RF energy in the first state during the initial output period, and so on. During output period, the target RF electrode subarray outputs RF energy in the first state. The second subarray determination module 20 determines the target RF electrode subarray in the output period when configuring the target RF electrode subarrays in the first output period to the output period as the historical RF electrode subarrays, until the target RF electrode subarrays have been determined as the historical RF electrode subarrays.

It can be understood that after the first subarray determination module 10 determines the target RF electrode subarray in the initial output period, the second subarray determination module 20 can repetitively execute the subarray selection unit and the second subarray determination unit in sequence based on the remaining RF electrode subarrays in the RF electrode array 50, so as to determine the target RF electrode subarrays in a plurality of new output periods at one time. Of course, it can be understood that the second subarray determination module 20 can also execute the subarray selection unit and the second subarray determination unit in sequence before the end of each current output period, to determine the target RF electrode subarray in the next output period, thereby obtaining the target RF electrode subarrays in a plurality of new output periods. That is, before the end of the current output period, the target RF electrode subarray in the next output period (if any) is determined.

In an embodiment, the second subarray determination unit is configured to determine the target RF electrode subarray in the new output period which includes at least two RF electrode subarrays that are not adjacent with each other from all nonadjacent RF electrode subarrays of the target RF electrode subarray in the current output period.

Similar to determining the target RF electrode subarray in the initial output period, for the new output period, in this embodiment, the target RF electrode subarray in the new output period which includes at least two RF electrode subarrays that are not adjacent with each other are determined, so that the time required to finish a treatment can be greatly reduced under the premise that the human body does not feel pain.

The control module 30 is configured to control the target RF electrode subarray in the current output period to be in the first state to output RF energy during the current output period, control the historical RF electrode subarray to be in the second state or the third state, and also control at least part of the remaining RF electrode subarrays in the RF electrode array 50 to be in the second state. An average power outputted by the RF electrode subarray in the first state is greater than an average power outputted by the RF electrode subarray in the second state, and the average power of the RF electrode subarray in the second state is greater than zero.

It should be understood that for the same RF electrode subarray, during the same time, the total RF energy outputted by the RF electrode subarray in the first state is much greater than the total RF energy outputted by the RF electrode subarray in the second state. That is, for each RF electrode subarray, when the RF electrode subarray outputs RF energy in the first state, it is a high energy output, and when the RF electrode subarray outputs RF energy in the second state, it is a low energy output. It can be seen that for each RF electrode subarray in this embodiment, the RF electrode subarray in the second state still outputs RF energy (in this case, the RF energy outputted by the RF electrode subarray in the second state is much less than the RF energy outputted by the RF electrode subarray in the first state per unit time), which can reduce the time required for the controller for controlling the RF electrode array to finish a treatment, thereby improving the treatment efficiency. That is to say, in this embodiment, two energy output modes are adopted for controlling the treatment process, which can reduce the treatment time while ensuring that the human body does not feel pain.

In addition, it should be understood that the current output period is the initial output period or the new output period. The target RF electrode subarray in the initial output period outputs RF energy in the first state when the RF electrode array 50 starts working. The target RF electrode subarray in each new output period will be switched to the first state to output RF energy only after the previous output period. Therefore, the historical RF electrode subarray gradually expands from not including any RF electrode subarray to including all RF electrode subarrays of the entire RF electrode array 50.

In an embodiment, for the current output period, if there is no historical RF electrode subarray (namely, during an initial output period), the control module 30 is configured to control the target RF electrode subarray in the current output period to be in the first state to output RF energy, and control at least part of the remaining RF electrode subarrays in the RF electrode array 50 to be in the second state. If there is a historical RF electrode subarray (namely, during a new output period), the control module 30 is configured to control the target RF electrode subarray in the current output period to be in the first state to output RF energy, control the historical RF electrode subarray to be in the second state or the third state, and also control at least part of the remaining RF electrode subarrays in the RF electrode array 50 to be in the second state.

For the control module 30, when it controls at least part of the remaining RF electrode subarrays in the RF electrode array 50 after excluding the target RF electrode subarray in the current output period and the historical RF electrode subarray to be in the second state, there are at least two situations. One situation is that the control module 30 controls all the remaining RF electrode subarrays in the RF electrode array 50 after excluding the target RF electrode subarray in the current output period and the historical RF electrode subarray to be in the second state. The other situation is that the control module 30 controls part of the remaining RF electrode subarrays in the RF electrode array 50 after excluding the target RF electrode subarray in the current output period and the historical RF electrode subarray to be in the second state, and controls another part of the remaining RF electrode subarrays to be in the third state.

In an embodiment, in the above second situation, when the control module 30 controls part of the remaining RF electrode subarrays in the RF electrode array 50 after excluding the target RF electrode subarray in the current output period and the historical RF electrode subarray to be in the second state, there are following schemes. The target RF electrode subarray in the next output period is controlled to be in the second state. It can be understood that this control method can preheat the treatment area to which the RF energy will be output in the next output period, and ensure the continuity of the treatment time of each treatment area, thereby shortening the treatment time and improving the treatment effect. Especially, not only the duration of the next output period in the first state can be reduced, but also the pain time, pain intensity and pain frequency of the same treatment area can be reduced. In an embodiment, for the current output period, the RF electrode subarrays (including the historical RF electrode subarray) after excluding the target RF electrode subarrays in the current output period (in the first state at this time) and the target RF electrode subarrays in the next output period (in the second state at this time) can be controlled to be in the third state.

In this embodiment, the third state is a zero-RF energy state or a no output state.

In this embodiment, in the current output period, for each historical RF electrode subarray, the historical RF electrode subarray is in the second state by default. When the RF energy received by the skin area treated by the historical RF electrode subarray has reached the energy required for treatment (that is, the received energy value is greater than or equal to the preset RF energy threshold), the historical RF electrode subarray is switched to the third state, and the historical RF electrode subarray is in the third state during the subsequent output period, so as to prevent the skin area treated by the historical RF electrode subarray from receiving too much RF energy and avoiding causing adverse effects on the human body.

In an embodiment, by precisely controlling the output power and output period duration of each RF electrode subarray, the state of each RF electrode subarray outputting RF energy can include only the first state and the second state during the entire treatment period, so as to achieve the maximum energy utilization rate.

In an embodiment, for each RF electrode subarray, the output power of the RF electrode subarray in the second state is less than the output power of the RF electrode subarray in the first state. In an embodiment, an output power of each RF electrode subarray in the second state is less than or equal to 40% of an output power of each RF electrode subarray in the first state.

It should be understood that, in this embodiment, for each RF electrode subarray, an increase in output power will lead to an increase in the outputted RF energy, while a decrease in output power will lead to a decrease in the outputted RF energy. It can be understood that this embodiment can avoid pain in the human body by realizing two energy output modes with different output powers, thereby realizing local area time-sharing treatment.

In another embodiment, for each RF electrode subarray, the output duty cycle of the RF electrode subarray in the second state is less than the output duty cycle of the RF electrode subarray in the first state. In an embodiment, the output duty cycle of each RF electrode subarray in the second state is less than or equal to 40% of the output duty cycle of each RF electrode subarray in the first state.

It should be understood that in this embodiment, within the preset unit time, for each RF electrode subarray, the output duty cycle refers to the ratio of the effective working time of the RF electrode subarray within the preset unit time to the total preset unit time.

In this embodiment, for each RF electrode subarray, when the output duty cycle is high, the effective working time of the RF electrode subarray in the current output period is relatively long, which means that the energy of the RF signal outputted by the RF electrode subarray will remain stable for a long time, thereby generating more RF energy. When the output duty cycle is low, the effective working time of the RF electrode subarray in the current output period is relatively short, which means that the energy of the RF signal outputted by the RF electrode subarray will be delivered in a shorter time, thereby generating less RF energy. That is to say, an increase in the output duty cycle will lead to an increase in the output RF energy, while a decrease in the output duty cycle will lead to a decrease in the output RF energy. It can be understood that, in this embodiment, two energy output modes can be realized with different output duty cycles, thereby realizing local area time-sharing treatment, which can avoid pain in the human body.

In an embodiment, the control module 30 includes a first control unit, an output accumulation unit, and a second control unit.

The first control unit is configured to control the target RF electrode subarray in the current output period to be in the first state to output RF energy, during the current output period, control the historical RF electrode subarray to be in the second state or the third state, and further control at least part of remaining RF electrode subarrays in the RF electrode array to be in the second state.

The output accumulation unit is configured to accumulate a cumulative RF energy value outputted by each RF electrode subarray from a first historical output period to a current output period.

The second control unit is configured to control each RF electrode subarray. The second control unit is configured to control each RF electrode subarray to be in a zero-RF energy state to stop outputting RF energy in response to that the cumulative RF energy is greater than or equal to a preset RF energy threshold.

In an embodiment, the first control unit is configured to control the target RF electrode subarray in the current output period to be in the first state to output RF energy. When the current output period is the initial output period, the first control unit also controls at least part of remaining RF electrode subarrays except the target RF electrode subarray to be in the second state. When the current output period is the new output period, the first control unit also controls the historical RF electrode subarray to be in the second state or the third state, and controls at least part of the remaining RF electrode subarrays in the RF electrode array 50 after excluding the target RF electrode subarray in the current output period and the historical RF electrode subarray to be in the second state.

It should be understood that the historical output period refers to the initial output period and the new output period. It can be understood that for each RF electrode subarray, in this embodiment, by counting the cumulative RF energy value outputted by each RF electrode subarray from the first historical output period to the current output period, and by controlling the RF electrode subarray to be in the zero-RF energy state according to the preset RF energy threshold, the excessive RF energy outputted to the same skin area of the human body can be avoided, thereby causing pain to the human body.

In an embodiment, the first control unit first control unit includes a temperature parameter acquisition subunit and an output power adjustment subunit.

The temperature parameter acquisition subunit is configured to acquire a real-time temperature parameter collected by a temperature sensor.

The output power adjustment subunit is configured to adjust a real-time output power of the target RF electrode subarray in the current output period according to the real-time temperature parameter.

It should be understood that when the target RF electrode subarray in the current output period outputs RF energy with a fixed power, the temperature of the skin area will rise, and when the temperature of human skin rises, it may cause pain. Based on this, as a specific implementation, the output power adjustment subunit is specifically configured to allow the RF energy output subunit to control the target RF electrode subarray in the current output period to output RF energy with the preset power when the real-time temperature parameter is less than the preset temperature threshold. The real-time temperature parameter refers to the temperature parameter of the skin area treated by the target RF electrode subarray in the current output period. When the real-time temperature parameter is greater than or equal to the preset temperature threshold, the real-time output power of the target RF electrode subarray in the current output period is adjusted, and the RF energy output subunit controls the target RF electrode subarray in the current output period to output RF energy with the real-time output power, so that the temperature of the skin area treated by the target RF electrode subarray in the current output period does not exceed the preset temperature threshold. The real-time output power is less than the preset power.

It can be understood that, in this embodiment, the real-time temperature parameter collected by the temperature sensor is obtained, and the temperature of the skin area treated by the target RF electrode subarray is controlled to not exceed the preset temperature threshold. That is, when the target RF electrode subarray outputs RF energy in the first state, the temperature of the skin area treated by the target RF electrode subarray first rises and then remains constant, thereby avoiding pain in the human body.

It can be seen that in this embodiment, during the process of using the RF electrode to deliver RF energy to the skin at a deep layer, by performing time-sharing treatment on each local area of the entire treatment skin, the instantaneous treatment area at each moment and the instantaneous pain can be reduced. In addition, at least part of the RF electrode subarray in the present application has outputted RF energy in the first state, and has also outputted RF energy in the second state. Through the energy accumulation of the low-power energy outputted in the second state, the time when the RF energy is outputted in the first state with a high power until the energy can achieve the treatment purpose will be partially reduced, and the overall treatment time is shortened, thereby solving the problem that when the target object of the RF energy outputted at each moment is the entire treatment area under the premise of the same total output power, the treatment time is long and it is easy to cause pain in the human body. Moreover, during the entire treatment process, the low RF energy outputted by the RF electrode subarray in the second state at low power or through other means can generate heat diffusion in the treatment area, thereby improving the treatment uniformity, and expanding the treatment area without increasing the pain sensation. In this way, the treatment effectiveness can be improved.

Based on the same inventive concept, as shown in FIG. 2, in the second aspect, the present application also provides an embodiment of an RF treatment device. The RF treatment device includes an RF generator 40, an RF electrode array 50 and a controller for controlling the RF electrode array as mentioned in the first aspect.

It should be noted that in the present application, the RF treatment device can be a noninvasive RF treatment device or an RF micro-needle treatment device. The noninvasive RF treatment device only needs to attach the RF electrode to the skin surface to release RF energy, and does not need to use micro-needles and other structures to pierce the skin epidermis. The RF micro-needle treatment device includes an RF micro-needle array. During the treatment process, the RF micro-needle array needs to pierce the skin epidermis and penetrate into the deep tissue of the skin, such as the dermis, subcutaneous fat layer or SMAS layer, to release RF energy.

In an embodiment, the RF generator 40 includes a plurality of separately controllable RF sub-generators, and each RF sub-generator is configured to control the target RF electrode subarray in different output periods. For each output period, the target RF electrode subarray in the output period is connected to the RF sub-generator in the current output period, so that the RF sub-generator provides energy for the target RF electrode subarray.

It can be understood that the RF generator 40 can separately supply power for the target RF electrode subarray that outputs RF energy simultaneously, which can improve the effect and safety of the RF treatment device.

In an embodiment, the RF treatment device has a monopolar mode. All RF electrodes in one RF electrode subarray have same electrical polarity in the monopolar mode.

In another embodiment, the RF treatment device has a bipolar mode. One RF electrode subarray includes at least two RF electrodes with opposite electrical polarities in the bipolar mode.

It can be understood that a better treatment effect can be obtained by selecting a suitable working mode according to the specific conditions and treatment needs of the patient.

To further facilitate understanding, a specific embodiment is further described in detail below.

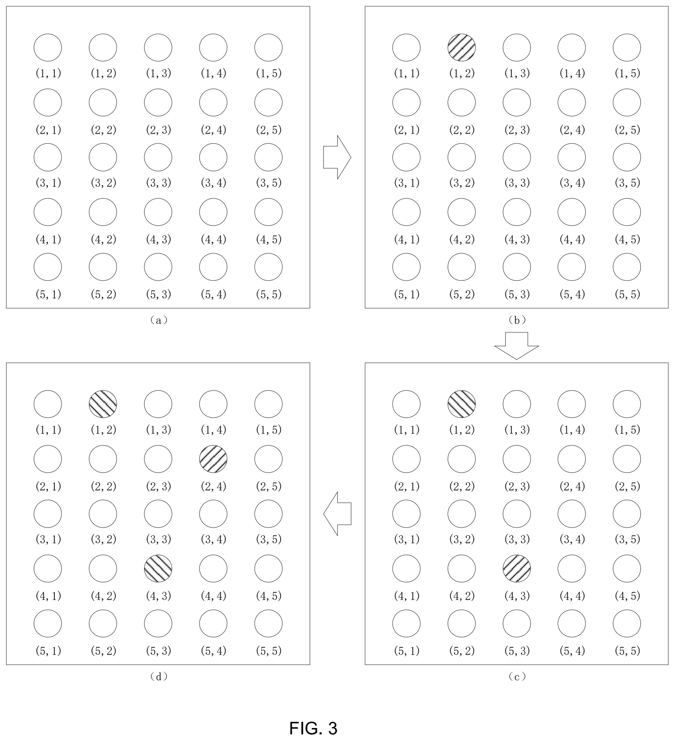

As shown in FIG. 3, FIG. 3 is a schematic structural diagram of an RF electrode array 50 in the monopolar mode. (a) of FIG. 3 is a schematic structural diagram of the RF electrode array 50 before starting. (b) of FIG. 3, (c) of FIG. 3, and (d) of FIG. 3 are schematic structural diagrams of the RF electrode array 50 in the first output period, the second output period, and the third output period, respectively.

As shown in FIG. 3, the RF electrode array 50 includes 25 RF electrode subarrays. In the monopolar mode, each RF electrode subarray includes an RF electrode. For ease description, each RF electrode subarray is named according to row and column coordinates. The names of each RF electrode subarray are as shown in FIG. 3, namely (1,1)to (5,5). For the RF electrode array 50 composed of these 25 RF electrode subarrays, 25 output periods are required to traverse.

During the treatment process, for the monopolar mode of this embodiment, a common electrode with opposite electrical polarities to the RF electrode array 50 is attached to other parts of the non-treatment area of the human body. In order to allow the RF electrode array 50 to deliver RF energy to deep skin tissues, such as the dermis and subcutaneous fat layer, a conductive channel is established between the RF electrode array 50 and the common electrode. By turning on the power switch of the RF electrode array 50, each RF electrode subarray emits RF energy, and RF current will be generated at the above-mentioned deep skin tissue to heat the tissue, thereby promoting collagen contraction and denaturation, starting collagen regeneration and reorganization, repairing and reshaping the skin. For the solution that the RF electrode is an RF microneedle, after the RF discharge is finished, the RF microneedle array is pulled out of the skin. Since the penetration of the microneedle temporarily opens the quick absorption channel of the skin, the repair product can be applied to the skin to enter the skin through the quick absorption channel of the skin, thereby playing a better recovery role.

Next, the key steps of traversing the RF electrode array 50 once in this embodiment are described in detail.

After the device is started, as shown in (b) of FIG. 3, the first subarray determination module 10 determines the target RF electrode subarray (1,2) in the initial output period from the RF electrode array 50, and the target RF electrode subarray (1,2) is represented by the slash in (b) of FIG. 3. The control module 30 controls the target RF electrode subarray (1,2) in the current output period (in this case, it is the initial output period or the first output period) to be in the first state to output RF energy, makes the target RF electrode subarray (1,2) maintain the first state to output RF energy in the current output period, and controls the remaining 24 RF electrode subarrays to be in the second state.

Before the second output period begins, the second subarray determination module 20 configures the target RF electrode subarray (1,2) as the historical RF electrode subarray, which is represented by the backslash in (c) of FIG. 3. The second subarray determination module 20 determines the target RF electrode subarray (4,3) in the new output period (the second output period) from the remaining RF electrode subarrays of the RF electrode array 50 after excluding the historical RF electrode subarray, and the target RF electrode subarray (4,3) is represented by the slash in (c) of FIG. 3.

Then, when the initial output period ends and the second output period begins, the control module 30 controls the target RF electrode subarray (4,3) to be in the first state to output RF energy, and controls the remaining RF electrode subarrays to be in the second state.

Before the third output period begins, the second subarray determination module 20 configures the target RF electrode subarray (4,3) as the historical RF electrode subarray, and determines the target RF electrode subarray (2,4) in the new output period (the third output period) from the remaining RF electrode subarrays of the RF electrode array 50 after excluding all the historical RF electrode subarrays (including (1,2) and (4,3), which are represented by the backslash in (d) of FIG. 3). The target RF electrode subarray (2,4) is represented by the slash in (d) of FIG. 3.

Then, when the second output period ends and the third output period begins, the control module 30 controls the target RF electrode subarray (2,4) to be in the first state to output RF energy, and controls the remaining RF electrode subarrays to be in the second state.

Similarly, the second subarray determination module 20 repetitively executes the operation of configuring the target RF electrode subarray as the historical RF electrode subarray, and determines the target RF electrode subarray in the new output period from the remaining RF electrode subarrays of the RF electrode array 50 after excluding all the historical RF electrode subarrays, until all of the RF electrode subarrays in the RF electrode array 50 have been determined as the historical RF electrode subarrays, which is considered to be a complete traversal.

For a single RF treatment, the RF electrode array 50 is usually only traversed once in one single tissue area. In some cases, the RF electrode array 50 can also be traversed a plurality of times in one single tissue area. For this method, after each traversal, all of the historical RF electrode subarrays need to be reset and refreshed.

In an embodiment, as shown in FIG. 3, the spatial positions of the target RF electrode subarrays in two adjacent output periods are not adjacent, and specifically, the function is performed by the subarray selection unit as mentioned above.

In a variant embodiment shown in FIG. 3, the difference between this embodiment and the above embodiment is that when the target RF electrode subarray outputs RF energy in the first state during the current output period, only part of the remaining RF electrode subarrays are controlled to be in the second state, that is, not all of the remaining RF electrode subarrays are controlled to be in the second state. For example, during the initial output period shown in (b) of FIG. 3, the target RF electrode subarray (1,2) is controlled to be in the first state, and a part of the remaining 24 RF electrode subarrays is controlled to be in the second state. In the second output period of (c) of FIG. 3, the target RF electrode subarray (4,3) is controlled to be in the first state, and part of the remaining 24 RF electrode subarrays are controlled to be in the second state.

In another variant embodiment shown in FIG. 3, the difference between this embodiment and the above embodiment is that, for the current output period, if the current output period has a next output period, the control module 30 not only controls the target RF electrode subarray in the current output period to be in the first state to output RF energy, and makes the target RF electrode subarray maintain the first state to output RF energy during the current output period, but also controls the target RF electrode subarray in the next output period to be in the second state. It can be seen that this variant embodiment is a special case of the above-mentioned variant embodiment. Taking FIG. 3 as an example, in the initial output period shown in (b) of FIG. 3, the target RF electrode subarray (1,2) is controlled to be in the first state, and the target RF electrode subarray (4,3) in the second output period is controlled to be in the second state. In the second output period shown in (c) of FIG. 3, the target RF electrode subarray (4,3) in the current output period is controlled to be in the first state, and the target RF electrode subarray (2,4) in the third output period is controlled to be in the second state. After excluding the RF electrode subarrays in the first state or the second state during the current output period, the remaining RF electrode subarrays may be in the third state, the zero-RF energy state or the no output state.

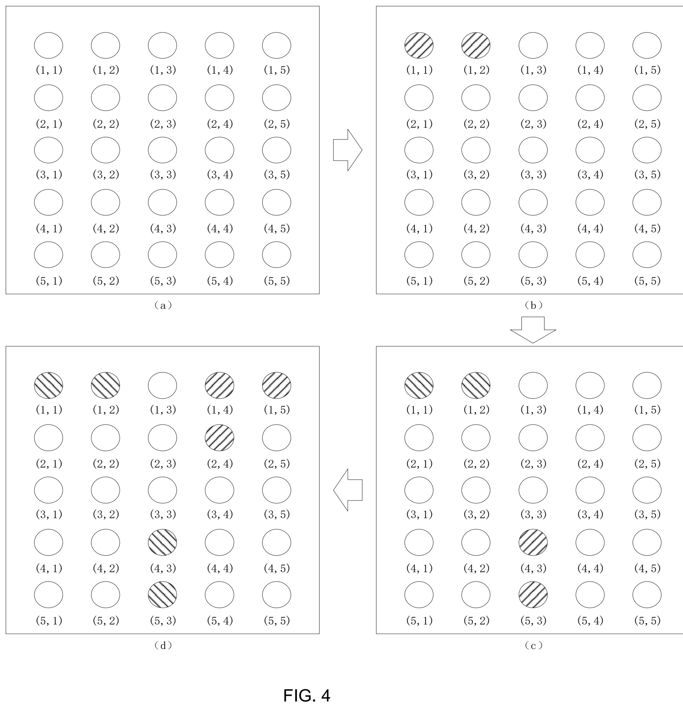

As shown in FIG. 4, FIG. 4 is a schematic structural diagram of the RF electrode array 50 in the bipolar mode. The same as FIG. 3 and its variant embodiment is that the target RF electrode subarray is represented by the slash, the historical RF electrode subarray is represented by the backslash, and the remaining RF electrode subarrays are represented by the blank. (a) of FIG. 4 is a schematic structural diagram of the RF electrode array 50 before it is started. (b) of FIG. 4, (c) of FIG. 4, and (d) of FIG. 4 are schematic structural diagrams of the RF electrode array 50 during the first output period, the second output period, and the third output period, respectively.

The main difference from the RF electrode array 50 in the monopolar mode shown in the embodiment of FIG. 3 and its variant embodiment is that, in the RF electrode array 50 in the bipolar mode of FIG. 4, the target RF electrode subarray includes at least two RF electrodes, and these two RF electrodes have different electrical polarities (not shown in the figure). That is, one electrical polarity is positive and one electrical polarity is negative, so as to form an RF current channel in the deep skin tissue between positive electrodes and negative electrodes, heat the tissue, and perform treatment.

In addition, in the embodiment shown in FIG. 4, the number of electrodes in the target RF electrode subarrays in different output periods is not necessarily the same. For example, in FIG. 4, the target RF electrode subarrays in the first output period include two RF electrodes, and the target RF electrode subarrays in the second output period include two RF electrodes, while the target RF electrode subarray in the third output period includes three RF electrodes (the electrical polarities of two RF electrodes are positive and the electrical polarity of one RF electrode is negative, or the electrical polarities of two RF electrodes are negative and the electrical polarity of one RF electrode is positive).

The above are only some embodiments of the present application, and do not limit the scope of the present application thereto. Under the concept of the present application, any equivalent structural transformation made according to the description and drawings of the present application, or direct/indirect application in other related technical fields shall fall within the claimed scope of the present application.

## Claims

1. A controller for controlling a radio frequency (RF) electrode array, applied to an RF treatment device, **characterized by** comprising: an RF generator; and the RF electrode array electrically connected to the RF generator; the RF electrode array comprising a plurality of RF electrode subarrays, and each RF electrode subarray comprising at least one RF electrode; the controller comprising:
a first subarray determination module, configured to determine a target RF electrode subarray from the RF electrode array in an initial output period;
a second subarray determination module, configured to repetitively execute an operation of determining the target RF electrode subarray as a historical RF electrode subarray, and determining the target RF electrode subarray in a new output period from remaining RF electrode subarrays after excluding all historical RF electrode subarrays until all RF electrode subarrays in the RF electrode array have been determined as historical RF electrode subarrays; and
a control module, configured to control the target RF electrode subarray in a current output period to be in a first state to output RF energy during the current output period, control the historical RF electrode subarray to be in a second state or a third state, and control at least part of remaining RF electrode subarrays in the RF electrode array to be in the second state,
wherein an average power outputted by the RF electrode subarray in the first state is greater than an average power outputted by the RF electrode subarray in the second state, and the average power of the RF electrode subarray in the second state is greater than zero.

2. The controller for controlling the RF electrode array according to claim 1, wherein the second subarray determination module comprises:
a subarray selection unit, configured to determine all of the RF electrode subarrays that are not adjacent to the target RF electrode subarray in the current output period from the remaining RF electrode subarrays after excluding all historical RF electrode subarrays;
a second subarray determination unit, configured to determine the target RF electrode subarray in the new output period from all of the RF electrode subarrays that are not adjacent to the target RF electrode subarray in the current output period; and
a repetitive control unit, configured to repetitively execute the subarray selection unit and the second subarray determination unit in sequence until all of the RF electrode subarrays in the RF electrode array have been determined as the historical RF electrode subarrays.

3. The controller for controlling the RF electrode array according to claim 1, wherein the control module is further configured to control the target RF electrode subarray to be in the second state in a next output period, during the current output period.

4. The controller for controlling the RF electrode array according to claim 1, wherein the control module comprises:
a first control unit, configured to control the target RF electrode subarray in the current output period to be switched in the first state to output RF energy during the current output period, and control the historical RF electrode subarray to be in the second state or the third state, and further control at least part of remaining RF electrode subarrays in the RF electrode array to be in the second state;
an output accumulation unit, configured to accumulate a cumulative RF energy outputted by each RF electrode subarray from a first historical output period to the current output period; and
a second control unit, configured to control each RF electrode subarray, wherein in response to that the cumulative RF energy value is greater than or equal to a preset RF energy threshold, the second control unit is configured to control the RF electrode subarray to be in a zero-RF energy state.

5. The controller for controlling the RF electrode array according to claim 4, wherein the first control unit comprises:
a temperature parameter acquisition subunit, configured to acquire a real-time temperature parameter collected by a temperature sensor; and
an output power adjustment subunit, configured to adjust a real-time output power of the target RF electrode subarray in the current output period according to the real-time temperature parameter.

6. The controller for controlling the RF electrode array according to any one of claims 1 to 5, wherein the target RF electrode subarray in the current output period comprises at least two RF electrode subarrays that are not adjacent with each other.

7. The controller for controlling the RF electrode array according to any one of claims 1 to 5, wherein each of the target RF electrode subarray has equal number of RF electrodes, or a difference between numbers of RF electrodes in each target RF electrode subarray is less than a preset number threshold.

8. The controller for controlling the RF electrode array according to any one of claims 1 to 5, wherein an output power of the RF electrode subarray in the second state is less than or equal to 40% of an output power of the RF electrode subarray in the first state.

9. The controller for controlling the RF electrode array according to any one of claims 1 to 5, wherein an output power of the RF electrode subarray in the second state is less than or equal to 40% of an output power of the RF electrode subarray in the first state.

10. An RF treatment device, **characterized in that**, the RF treatment device comprises an RF electrode array and an RF generator, and the controller for controlling the RF electrode array according to claim 1.

11. The RF treatment device according to claim 10, wherein the RF generator comprises a plurality of separately controllable RF sub-generators, and each RF sub-generator is configured to control the target RF electrode subarray in different output periods.

12. The RF treatment device according to claim 10, wherein the RF treatment device comprises a monopolar mode, and all RF electrodes in one RF electrode subarray have same electrical polarities in the monopolar mode.

13. The RF treatment device according to claim 10, wherein the RF treatment device comprises a bipolar mode, and one RF electrode subarray comprises at least two RF electrodes with opposite electrical polarities in the bipolar mode.
